# EUROPEAN PATENT APPLICATION

(11) **EP 4 756 815 A1**
(43) Date of publication of application: **10.06.2026**
(21) Application number: 24849027.8
(22) Date of filing: 24.07.2024
(51) Int. Cl.: G16B 40/20, C12M 1/00, C12M 1/34

(54) **CELL CHARACTERISTIC PROPERTY PREDICTION DEVICE, METHOD FOR OPERATING CELL CHARACTERISTIC PROPERTY PREDICTION DEVICE, AND OPERATION PROGRAM FOR CELL CHARACTERISTIC PROPERTY PREDICTION DEVICE**

(30) Priority: 31.07.2023 JP 2023125053
(71) Applicant: FUJIFILM Corporation, Tokyo 106-8620 (JP)
(72) Inventor: NAGASE, Masaya, Ashigarakami-gun, Kanagawa 258-8577 (JP); UMEKAWA, Masao, Ashigarakami-gun, Kanagawa 258-8577 (JP); SUZUKI, Takafumi, Ashigarakami-gun, Kanagawa 258-8577 (JP)
(74) Representative: Hoffmann Eitle
(86) International application number: PCT/JP2024/026517
(87) International publication number: WO 2025/028372

(57) **Abstract**

A cell characteristic prediction apparatus that predicts characteristics of a target cell that is a cell obtained through monoclonalization from a host cell population that is a collection of host cells having a plurality of different subtypes, the cell characteristic prediction apparatus including a processor, in which the processor is configured to: acquire subtype information representing a subtype of the target cell derived from a subtype of the host cell, and perform prediction depending on the subtype information.

## Description

### BACKGROUND OF THE INVENTION

### 1. Field of the Invention

The technology of the present disclosure relates to a cell characteristic prediction apparatus, an operation method for the cell characteristic prediction apparatus, and an operation program for the cell characteristic prediction apparatus.

### 2. Description of the Related Art

At present, cell culture is actively performed for antibody-producing cells involved in the manufacture of an antibody pharmaceutical, as well as for cells such as cardiomyocytes and nerve cells induced to differentiate from induced pluripotent stem (iPS) cells. In such a field of cell culture, various techniques have been proposed to predict characteristics of cells by analyzing various types of data related to the cells using a computer, without actually performing long-term tests.

For example, JP2022-550083A describes a technique in which karyotype analysis is performed on an antibody-producing cell (referred to as a clone cell in JP2022-550083A) into which an antibody gene has been integrated and which have been monoclonalized (single-cell cloned), to derive, as a characteristic of the cell, a genome instability value of the antibody-producing cells and to select an antibody-producing cell that stably produces an antibody based on the genome instability value.

In addition, JP2022-533003A describes a technique in which a first attribute value of the antibody-producing cell measured by an optical-electrical cell line generation and analysis system and a second attribute value of the antibody-producing cell measured in a cell pool screening stage are analyzed using a machine learning-based regression estimator, to predict a value of a product quality attribute of an antibody-producing cell as a characteristic of a cell.

### SUMMARY OF THE INVENTION

Some cells have a plurality of different subtypes. The characteristics of the cell are greatly affected by the subtype. However, in the techniques described in JP2022-550083A and JP2022-533003A, the subtype is not considered in the prediction of the characteristics of the cell. Therefore, there is a possibility that the prediction accuracy of the characteristics of the cell is not sufficient.

One embodiment according to the technology of the present disclosure provides a cell characteristic prediction apparatus, an operation method for the cell characteristic prediction apparatus, and an operation program for the cell characteristic prediction apparatus, which can improve prediction accuracy of characteristics of a cell.

A cell characteristic prediction apparatus of the present disclosure is a cell characteristic prediction apparatus that predicts characteristics of a target cell that is a cell obtained through monoclonalization from a host cell population that is a collection of host cells having a plurality of different subtypes, the cell characteristic prediction apparatus including a processor, in which the processor is configured to: acquire subtype information representing a subtype of the target cell derived from a subtype of the host cell, and perform prediction depending on the subtype information.

It is preferable that the processor is configured to acquire target cell information related to the target cell, classify the subtype of the target cell based on the target cell information, and acquire a classification result of the subtype of the target cell as the subtype information.

It is preferable that the processor is configured to input the target cell information to a first machine learning model, and cause the first machine learning model to output the classification result of the subtype.

It is preferable that the target cell information includes at least one of gene data of the target cell, culture data of the target cell, or morphological data of the target cell.

It is preferable that the processor acquires target cell information related to the target cell, and predicts the characteristics of the target cell based on the target cell information.

It is preferable that the processor is configured to input the target cell information to a second machine learning model, and cause the second machine learning model to output a prediction result of the characteristics of the target cell.

It is preferable that the target cell information includes at least one of gene data of the target cell, culture data of the target cell, or morphological data of the target cell.

A plurality of tools for predicting the characteristics of the target cell are provided, and it is preferable that the processor is configured to select a matching tool adapted to the subtype information from among the plurality of tools, and perform the prediction using the matching tool.

It is preferable that the processor is configured to present a prediction result of the characteristics of the target cell to a user.

It is preferable that the processor is configured to present the subtype information to the user.

It is preferable that the processor is configured to present a reliability degree of the prediction result to the user.

It is preferable that the processor is configured to determine a subsequent culture condition of the target cell based on at least one of the subtype information or a prediction result of the characteristics of the target cell.

It is preferable that the host cell is a mammalian-derived cell.

It is preferable that the target cell is a cell that produces a substance that serves as an active ingredient of a biopharmaceutical, and the processor is configured to predict production stability of the substance as the characteristics of the target cell.

It is preferable that the substance is an antibody.

It is preferable that the processor is configured to predict a culture condition of the target cell as the characteristics of the target cell.

An operation method for a cell characteristic prediction apparatus according to the present disclosure is an operation method for a cell characteristic prediction apparatus that predicts characteristics of a target cell that is obtained through monoclonalization from a host cell population that is a collection of host cells having a plurality of different subtypes, the operation method including acquiring subtype information representing a subtype of the target cell derived from a subtype of the host cell, and performing the prediction depending on the subtype information.

An operation program for a cell characteristic prediction apparatus according to the present disclosure is an operation program for a cell characteristic prediction apparatus that predicts characteristics of a target cell that is obtained through monoclonalization from a host cell population that is a collection of host cells having a plurality of different subtypes, the operation program causing a computer to execute a process including acquiring subtype information representing a subtype of the target cell derived from a subtype of the host cell, and performing the prediction depending on the subtype information.

According to the technology of the present disclosure, it is possible to provide a cell characteristic prediction apparatus, an operation method for the cell characteristic prediction apparatus, and an operation program for the cell characteristic prediction apparatus, which can improve prediction accuracy of characteristics of a cell.

### BRIEF DESCRIPTION OF THE DRAWINGS

FIG. 1 is a diagram showing an outline of a manufacturing step of an antibody pharmaceutical.
FIG. 2 is a diagram showing a detailed procedure of a clone generation step.
FIG. 3 is a diagram showing a detailed procedure of a clone generation step.
FIG. 4 is a diagram showing a production stability prediction server and a user terminal.
FIG. 5 is a diagram showing an antibody-producing cell information set.
FIG. 6 is a block diagram showing a computer constituting the production stability prediction server and the user terminal.
FIG. 7 is a block diagram showing a processing unit of a CPU of the production stability prediction server.
FIG. 8 is a diagram showing processing of a classification unit.
FIG. 9 is a diagram showing a prediction model constituting a prediction model set.
FIG. 10 is a diagram showing a state in which a prediction model adapted to a classification result is selected.
FIG. 11 is a diagram showing processing of a prediction unit.
FIG. 12 is a block diagram showing a processing unit of a CPU of the user terminal.
FIG. 13 is a diagram showing an information input screen.
FIG. 14 is a diagram showing a prediction result display screen.
FIG. 15 is a flowchart showing a processing procedure of the production stability prediction server.
FIG. 16 is a diagram showing a prediction result display screen of a second embodiment.
FIG. 17 is a table showing a reliability degree of a prediction result of each prediction model.
FIG. 18 is a diagram showing a third embodiment in which a culture condition is determined based on a subtype.
FIG. 19 is a diagram showing a fourth embodiment in which a culture condition of an antibody-producing cell is predicted.
FIG. 20 is a performance comparison table between a prediction model of a comparative example and a prediction model of an example.

### DESCRIPTION OF THE PREFERRED EMBODIMENTS

### [First embodiment]

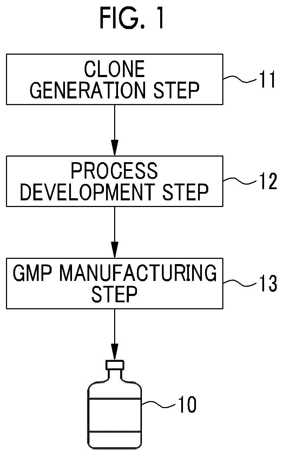

As shown in FIG. 1 as an example, an antibody pharmaceutical 10 is manufactured through a clone generation step 11, a process development step 12, and a good manufacturing practice (GMP) manufacturing step 13. The antibody pharmaceutical 10 is an example of a "biopharmaceutical" according to the technology of the present disclosure.

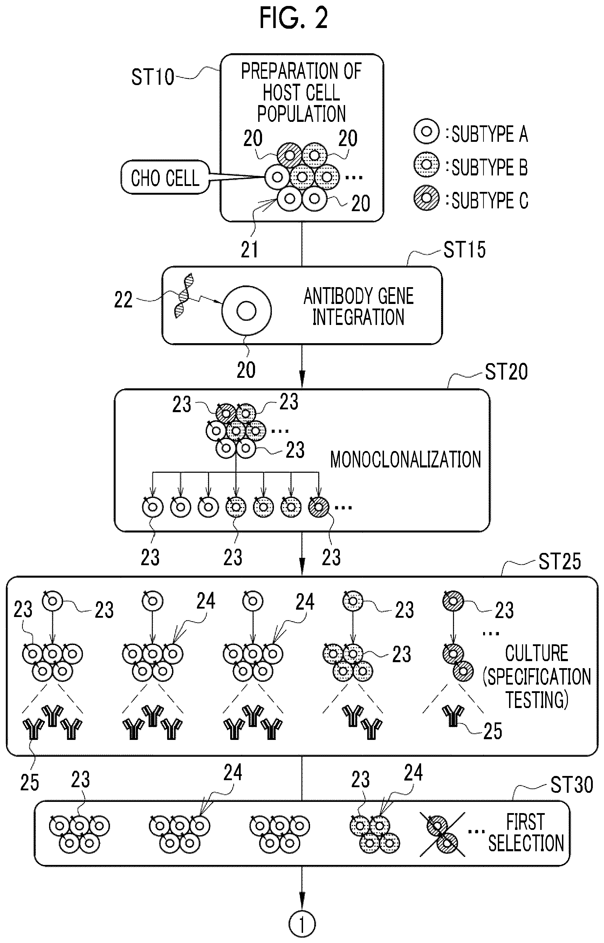

The clone generation step 11 is a step of generating a plurality of clones 24 of an antibody-producing cell 23 that produces an antibody 25 (all shown in FIG. 2), and selecting a clone 24 having excellent characteristics from among the plurality of clones 24. Here, the clone 24 refers to a population of genetically identical antibody-producing cells 23. The antibody-producing cell 23 is an example of a "target cell" and a "cell that produces a substance that is an active ingredient of a biopharmaceutical" according to the technology of the present disclosure. In addition, the antibody 25 is an example of a "substance that is an active ingredient of a biopharmaceutical" according to the technology of the present disclosure.

The process development step 12 is a step of developing a culture condition and a purification condition in the GMP manufacturing step 13 using the selected clone 24. The GMP manufacturing step 13 is a step of obtaining the antibody pharmaceutical 10 by causing the clone 24 to produce the antibody 25 and purifying and formulating the antibody 25 under the culture and purification conditions developed in the process development step 12. The technology of the present disclosure relates to the clone generation step 11 among the clone generation step 11, the process development step 12, and the GMP manufacturing step 13. Therefore, a detailed procedure of the clone generation step 11 will be described below.

As shown in FIG. 2 as an example, the clone generation step 11 starts with preparing a host cell population 21 that is a collection of a plurality of host cells 20 (Step ST10). The host cell 20 is a vertebrate-derived cell, more specifically, a mammal-derived cell, and is here a Chinese hamster ovary (CHO) cell. The host cell 20 has a congenital or acquired heterogeneity, and has three different subtypes of a subtype A, a subtype B, and a subtype C. The number of the host cells 20 is large for the subtypes A and B, and the subtype C is rare compared to the subtypes A and B. The host cell 20 is an example of a "host cell" according to the technology of the present disclosure, and the host cell population 21 is an example of a "host cell population" according to the technology of the present disclosure. It is noted that the acquired heterogeneity may be intentionally generated or may be naturally generated during culture.

The subtypes are different in, for example, genetic information. Alternatively, the subtypes may have different characteristics such as an expression level of ribonucleic acid (RNA), a size, morphology, and a proliferation rate. The subtypes may represent a plurality of similar subtypes, such as the subtype A including subtypes A1 and A2 and the subtype B including subtypes B1, B2, and B3. In addition, the number of the subtypes may be imbalanced as described above, or may be substantially the same.

It may be identified which subtypes are present in the host cell 20 by performing single-cell analysis on the host cell 20, and performing PCA-based clustering which is principal component analysis (PCA) combined with cluster analysis, or the like. Alternatively, a plurality of clones 24 of the antibody-producing cell 23 may be generated, and it may be identified which subtypes are present in the host cell 20 based on common features (RNA, single nucleotide polymorphism (SNP), copy number variation (CNV), or the like) of the clones 24 having different any measurement data such as a gene expression level and a proliferation rate.

Next, the antibody gene 22 is integrated into each of the host cells 20 constituting the host cell population 21 (Step ST15). In this way, the antibody-producing cell 23 is generated from the host cell 20. Subsequently, the plurality of antibody-producing cells 23 are monoclonalized one by one (Step ST20).

The monoclonalized antibody-producing cells 23 are cultured to produce a plurality of clones 24. Then, the antibody 25 is produced in each of the plurality of clones 24 (Step ST25). Step ST25 is specification testing performed to confirm an antibody production ability of each clone 24, and the period thereof is about 2 weeks.

The antibody production ability of each clone 24 is not constant, and there are clones having a high antibody production ability and clones having a low antibody production ability. Among these, there is a clone 24 that hardly produces the antibody 25. Therefore, after the specification testing of Step ST25 is completed, first selection is performed to select the clone 24 having a numerical value indicating the antibody production ability equal to or more than a preset threshold value (Step ST30). In other words, the first selection is work of excluding the clone 24 having a relatively low antibody production ability. The numerical value indicating the antibody production ability is, for example, an antibody production amount per unit time during the specification testing period.

Thereafter, in the related art, a test (production stability test) for confirming the production stability of the antibody 25 of the clone 24 is performed over a specified period, for example, 2 to 3 months. Therefore, it has been a hindrance to the manufacturing of the antibody pharmaceutical 10.

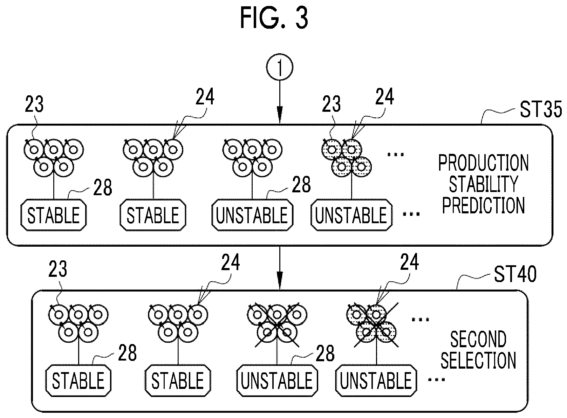

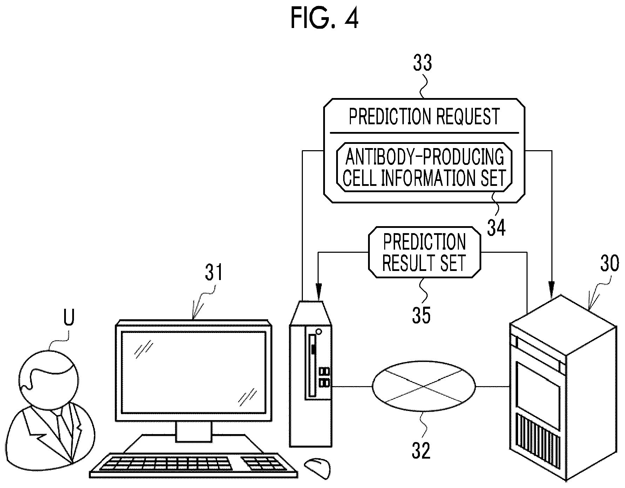

Therefore, in the technology of the present disclosure, as shown in Step ST35 of FIG. 3 as an example, the production stability of the antibody 25 of the clone 24 (antibody-producing cell 23) is predicted (hereinafter, referred to as production stability prediction) by analyzing various types of data related to the clone 24 (antibody-producing cell 23) using a computer, without actually performing the production stability test over 2 to 3 months. Then, a prediction result 28 of the production stability prediction is presented to a user U (see FIG. 4). It is noted that the start point of the specified period is a start point of the specification testing of Step ST25 or an end point of the first selection of Step ST30. The specified period may be a period in units of months such as 2 to 3 months described above, or may be a period until a predetermined number of subcultures are performed. Alternatively, the specified period may be determined based on a proliferation ability of the antibody-producing cell 23, or may be determined based on a period in which the actual culture is performed in the GMP manufacturing step 13. The production stability of the antibody 25 is an example of "characteristics of a target cell" according to the technology of the present disclosure.

The production stability is determined based on a degree of change in the amount of the antibody 25 produced by the clone 24, that is, an antibody production amount, over the specified period. Specifically, the degree of change in the antibody production amount over the specified period is a rate of increase or decrease in the antibody production amount at the start point and the end point of the specified period. The rate of increase or decrease in the antibody production amount is obtained by dividing the antibody production amount at the end point of the specified period by the antibody production amount at the start point. Since the antibody production amount is decreased in many cases, the rate of increase or decrease in the antibody production amount is rarely 100% or more, and is usually less than 100%. The production stability is stable in a case where the rate of increase or decrease in the antibody production amount is equal to or more than a preset threshold value. On the other hand, the production stability is unstable in a case where the rate of increase or decrease in the antibody production amount is lower than the threshold value. The threshold value is, for example, 70% or 80%.

After the production stability prediction of Step ST35, second selection is performed to select the clone 24 predicted to have stable production stability (Step ST40). In other words, the second selection is work of excluding the clone 24 predicted to have unstable production stability. The clone 24 selected in the second selection is used in the subsequent process development step 12 and GMP manufacturing step 13.

As shown in FIG. 4 as an example, a production stability prediction server 30 is connected to a user terminal 31 via a network 32. The production stability prediction server 30 is a server that performs the production stability prediction of Step ST35 shown in FIG. 3, and is an example of a "cell characteristic prediction apparatus" according to the technology of the present disclosure. The user terminal 31 is installed in a pharmaceutical company that develops a biopharmaceutical or an institution that receives a development business of a biopharmaceutical from the pharmaceutical company, that is, a contract research organization (CRO). The user terminal 31 is operated by the user U who is involved in the development of the biopharmaceutical in the pharmaceutical company or the contract research organization. The network 32 is, for example, a wide area network (WAN) such as the Internet or a public communication network. In FIG. 4, only one user terminal 31 is connected to the production stability prediction server 30, but in practice, a plurality of user terminals 31 of a plurality of pharmaceutical companies or contract research organizations are connected to the production stability prediction server 30.

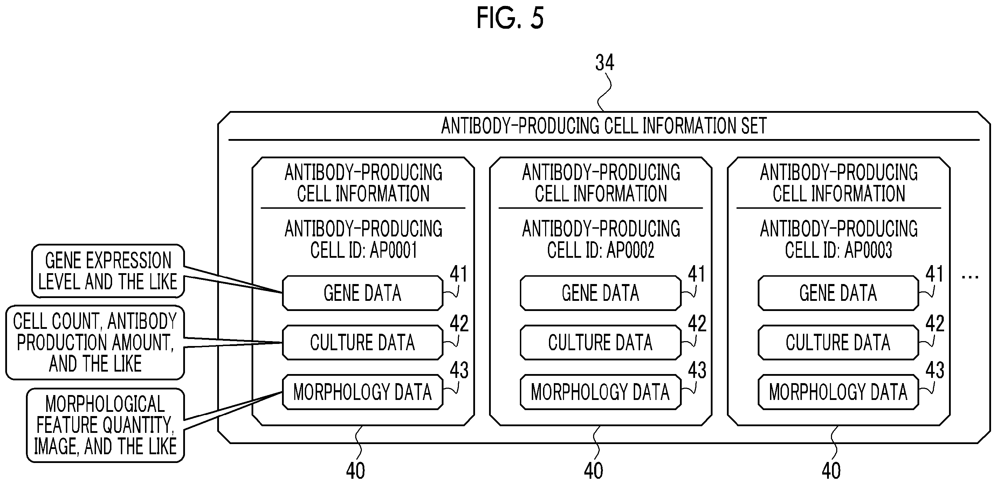

The user terminal 31 transmits a prediction request 33 to the production stability prediction server 30. The prediction request 33 is a request for the production stability prediction server 30 to perform the production stability prediction. The prediction request 33 includes an antibody-producing cell information set 34. The antibody-producing cell information set 34 is a collection of antibody-producing cell information 40 (see FIG. 5) related to the antibody-producing cell 23 constituting each clone 24 after the first selection. The antibody-producing cell information 40 is an example of "target cell information" according to the technology of the present disclosure. Although not shown, the prediction request 33 also includes a terminal identification data (ID) or the like for uniquely identifying the user terminal 31 which is a transmission source of the prediction request 33.

In a case in which the prediction request 33 is received, the production stability prediction server 30 performs the production stability prediction for the antibody-producing cell 23 of each clone 24 after the first selection. Then, a prediction result set 35 that is a collection of the prediction results 28 is delivered to the user terminal 31 that is the transmission source of the prediction request 33. In a case in which the prediction result set 35 is received, the user terminal 31 provides the prediction result set 35 for viewing by the user U.

As shown in FIG. 5 as an example, the antibody-producing cell information 40 includes an antibody-producing cell ID for uniquely identifying the antibody-producing cell 23 constituting each clone 24, and gene data 41 of the antibody-producing cell 23, culture data 42 of the antibody-producing cell 23, and morphological data 43 of the antibody-producing cell 23. The gene data 41 is a gene expression level of the antibody-producing cell 23 obtained by RNA sequence analysis or quantitative polymerase chain reaction (qPCR) analysis, and the like. The gene expression level is a count value that takes a positive integer, and can be used after logarithmic conversion. The culture data 42 is a cell count, an antibody production amount, and the like of the antibody-producing cell 23 measured after the specification testing of Step ST25 shown in FIG. 2. The morphological data 43 is a morphological feature quantity of the antibody-producing cell 23 measured after the specification testing, an image of the antibody-producing cell 23 captured after the specification testing, and the like. The morphological feature quantity is, for example, a representative value of a major axis, a minor axis, an area, and/or the like of the antibody-producing cell 23. In a case of the image, a representative value of pixel values may be used as the morphological data instead of the image itself. The representative value is an average value, a maximum value, a minimum value, a mode value, a median value, and/or the like.

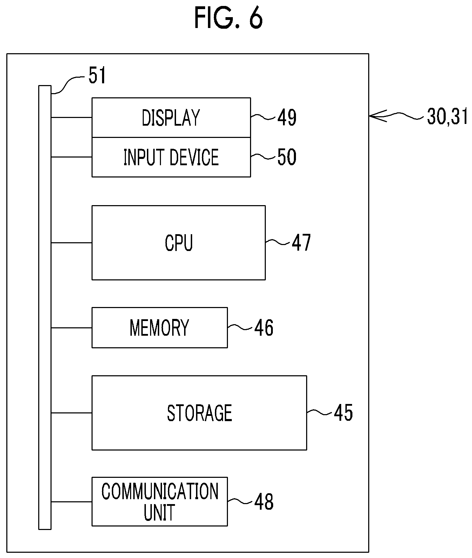

As shown in FIG. 6 as an example, the computers constituting the production stability prediction server 30 and the user terminal 31 basically have the same configuration, and comprise a storage 45, a memory 46, a central processing unit (CPU) 47, a communication unit 48, a display 49, and an input device 50. These units are connected to each other via a busline 51.

The storage 45 is a hard disk drive that is built in the computers constituting the production stability prediction server 30 and the user terminal 31 or connected thereto through a cable or a network. Alternatively, the storage 45 is a disk array in which a plurality of hard disk drives are connected together. The storage 45 stores a control program such as an operating system, various application programs (hereinafter, referred to as an application program (AP)), various types of data associated with these programs, and the like. It should be noted that a solid state drive may be used instead of the hard disk drive.

The memory 46 is a work memory for the CPU 47 to execute processing. The CPU 47 loads the program stored in the storage 45 into the memory 46 to execute the processing in accordance with the program. Thus, the CPU 47 collectively controls the respective units of the computer. The CPU 47 is an example of a "processor" according to the technology of the present disclosure. The memory 46 may be built in the CPU 47.

The communication unit 48 is a network interface that performs control of transmitting various types of information via a network 32 and the like. The display 49 displays various screens. The various screens have an operation function by a graphical user interface (GUI). The computers constituting the production stability prediction server 30 and the user terminal 31 receive input of an operation instruction from the input device 50 through various screens. The input device 50 is, for example, a keyboard, a mouse, a touch panel, and a microphone for voice input.

In the following description, the respective units (the storage 45 and the CPU 47) of the computer constituting the production stability prediction server 30 are distinguished by adding a subscript "A" to the reference numerals thereof, and the respective units (the storage 45, the CPU 47, the display 49, and the input device 50) of the computer constituting the user terminal 31 are distinguished by adding a subscript "B" to the reference numerals thereof.

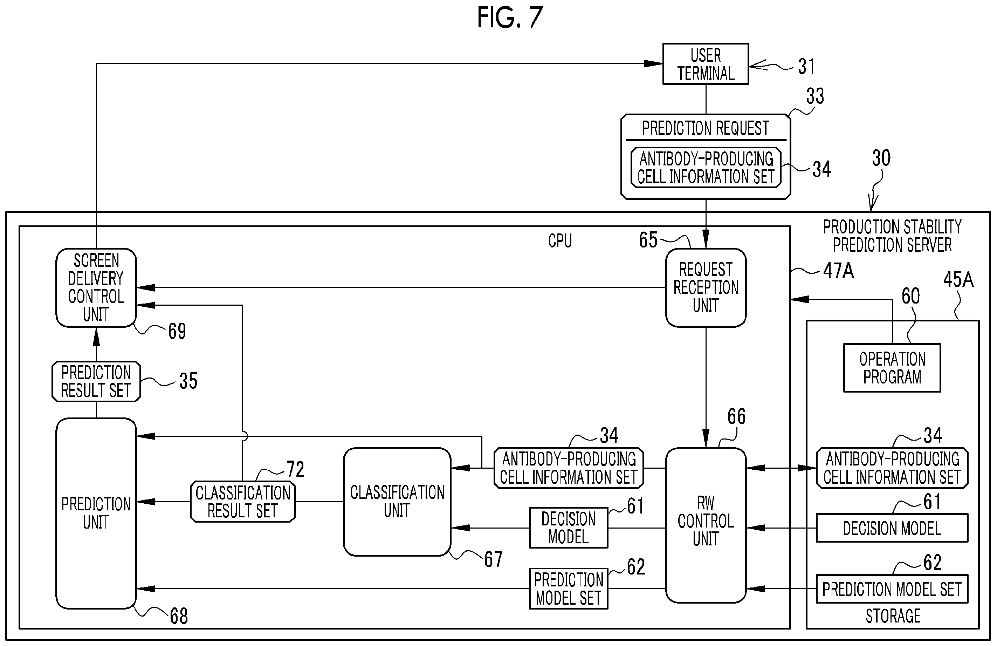

As shown in FIG. 7 as example, an operation program 60 is stored in the storage 45A of the production stability prediction server 30. The operation program 60 is an AP for causing the computer to function as the production stability prediction server 30. That is, the operation program 60 is an example of an "operation program of a cell characteristic prediction apparatus" according to the technology of the present disclosure. The storage 45A also stores the antibody-producing cell information set 34, a classification model 61, a prediction model set 62, and the like.

In a case in which the operation program 60 is activated, the CPU 47A of the computer constituting the production stability prediction server 30 functions as a request reception unit 65, an RW control unit 66, a classification unit 67, a prediction unit 68, and a screen delivery control unit 69 in cooperation with the memory 46 and the like.

The request reception unit 65 receives various requests from the user terminal 31. In particular, the request reception unit 65 receives the prediction request 33 from the user terminal 31. As described above, the prediction request 33 includes an antibody-producing cell information set 34. Then, the antibody-producing cell information set 34 is a collection of a plurality of pieces of antibody-producing cell information 40. Therefore, the request reception unit 65 acquires the antibody-producing cell information 40 by receiving the prediction request 33. In a case where the request reception unit 65 receives the prediction request 33, the request reception unit 65 outputs the antibody-producing cell information set 34 included in the prediction request 33 to the RW control unit 66. In addition, the request reception unit 65 outputs the terminal ID of the user terminal 31 included in the prediction request 33 to the screen delivery control unit 69.

The RW control unit 66 controls storage of various types of data in the storage 45A and readout of various types of data from the storage 45A. For example, the RW control unit 66 stores the antibody-producing cell information set 34 from the request reception unit 65 in the storage 45A. In addition, the RW control unit 66 reads out the antibody-producing cell information set 34 from the storage 45A, and outputs the readout antibody-producing cell information set 34 to the classification unit 67 and the prediction unit 68. In addition, the RW control unit 66 reads out the classification model 61 from the storage 45A, and outputs the readout classification model 61 to the classification unit 67. The RW control unit 66 reads out the prediction model set 62 from the storage 45A and outputs the readout prediction model set 62 to the prediction unit 68.

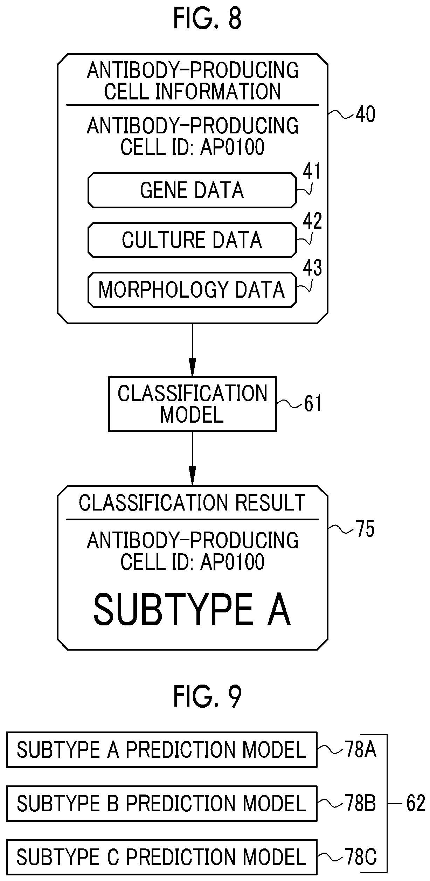

The classification unit 67 classifies the subtype of the antibody-producing cell 23 constituting each clone 24 by using the classification model 61. The subtype of the antibody-producing cell 23 is derived from the subtype of the original host cell 20. The classification unit 67 outputs a classification result set 72 that is a collection of classification results 75 (see FIG. 8) of the subtypes to the prediction unit 68 and the screen delivery control unit 69.

The prediction unit 68 performs the production stability prediction of each clone 24 by using the prediction model set 62. The prediction unit 68 outputs a prediction result set 35 that is a collection of the prediction results 28 of the production stability prediction of each clone 24 to the screen delivery control unit 69.

The screen delivery control unit 69 controls delivery of various screens to the user terminal 31. Specifically, the screen delivery control unit 69 delivers output of the various screens to the user terminal 31 that is a transmission source of the various requests, in the form of screen data for web delivery created using a markup language such as extensible markup language (XML). In this case, the screen delivery control unit 69 specifies the user terminal 31 that is the transmission source of various requests based on the terminal ID from the request reception unit 65. Note that, instead of XML, another data description language, such as JavaScript (registered trademark) Object Notation (JSON), may be used.

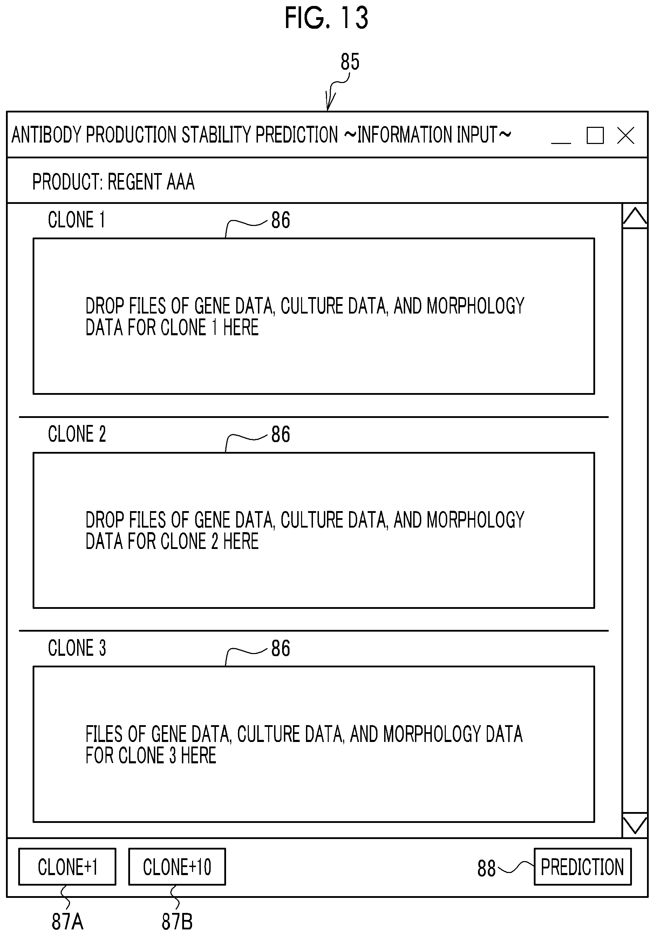

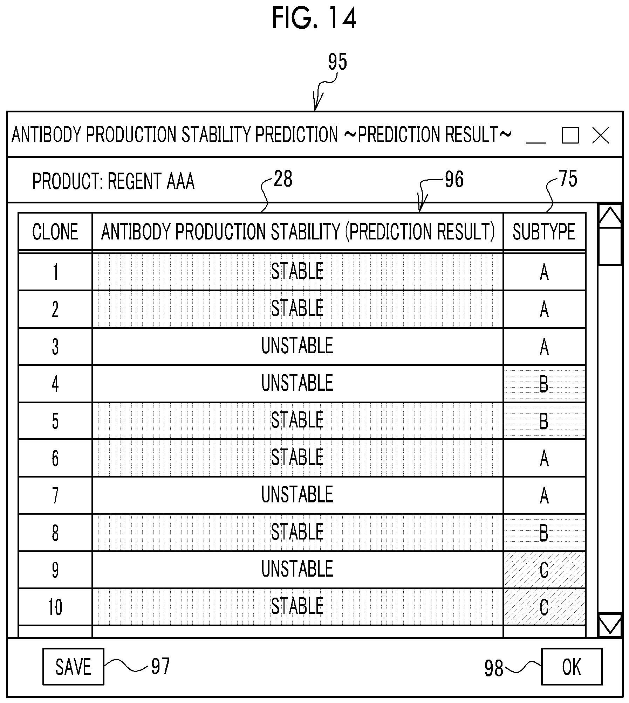

Various screens include an information input screen 85 (see FIG. 13) for inputting the antibody-producing cell information 40, a prediction result display screen 95 (see FIG. 14) for displaying the prediction result 28, and the like. An instruction reception unit that receives various operation instructions from the input device 50, and the like are also constructed in the CPU 47A, in addition to each of the processing units 65 to 69.

As shown in FIG. 8 as an example, the classification unit 67 inputs the antibody-producing cell information 40 (the gene data 41, the culture data 42, and the morphological data 43) to the classification model 61, and causes the classification model 61 to output a classification result 75. The classification result 75 includes the antibody-producing cell ID and the subtype of the antibody-producing cell 23. That is, the classification result 75 is an example of "subtype information" according to the technology of the present disclosure.

The classification model 61 is a machine learning model that has been trained to classify the subtype of the antibody-producing cell 23 based on the antibody-producing cell information 40. The training data of the classification model 61 is a pair of the antibody-producing cell information 40 of the antibody-producing cell 23 generated in the past and the subtype (correct answer data) clarified by detailed genetic analysis or the like of the antibody-producing cell 23. It is noted that by inputting a plurality of elements constituting the antibody-producing cell information 40 excluded one or a plurality of the elements to the classification model 61, outputting the classification result 75 for training from the classification model 61, and comparing the classification result in a case where the element is excluded with the classification result 75 in a case where the element is not excluded, a contribution degree of the excluded element to the classification result 75 may be calculated, and the element with high contribution degree may be narrowed down and used as the element finally input to the classification model 61. For example, for the gene data 41 of the antibody-producing cell information 40, the gene expression level of a few to several tens of genes (so-called marker genes) having a high contribution degree may be narrowed down to the gene expression level finally input to the classification model 61 from the gene expression levels of hundreds of genes. In this case, the genes that exhibit statistically significant differences in the gene expression levels among the subtypes may be first narrowed down, and further narrowing down may be performed based on the contribution degree.

The classification model 61 outputs, for example, a probability that the antibody-producing cell 23 is the subtype A, a probability that the antibody-producing cell 23 is the subtype B, and a probability that the antibody-producing cell 23 is the subtype C, and classifies the subtype having the highest probability as the subtype of the antibody-producing cell 23. The probability that the antibody-producing cell 23 is the subtype A, the probability that the antibody-producing cell 23 is the subtype B, and the probability that the antibody-producing cell 23 is the subtype C sum to 100%. The classification model 61 is an example of a "first machine learning model" according to the technology of the present disclosure.

As shown in FIG. 9 as an example, the prediction model set 62 is composed of three prediction models 78, that is, a subtype A prediction model 78A, a subtype B prediction model 78B, and a subtype C prediction model 78C. The subtype A prediction model 78A, the subtype B prediction model 78B, and the subtype C prediction model 78C are models that perform the production stability prediction of the antibody-producing cell 23 of the subtype A, the antibody-producing cell 23 of the subtype B, and the antibody-producing cell 23 of the subtype C. The subtype A prediction model 78A, the subtype B prediction model 78B, and the subtype C prediction model 78C are examples of a "second machine learning model" and "a plurality of tools" according to the technology of the present disclosure.

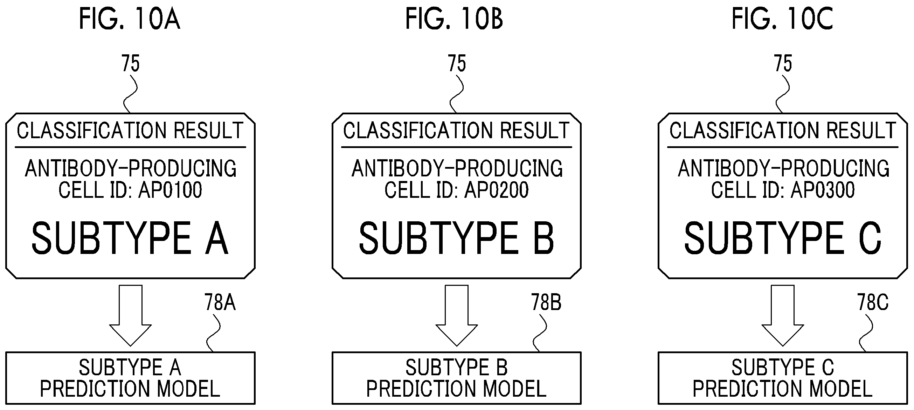

As shown in FIG. 10A as an example, in a case where the subtype of the antibody-producing cell 23 based on the classification result 75 is the subtype A, the prediction unit 68 selects the subtype A prediction model 78A. As shown in FIG. 10B, in a case where the subtype of the antibody-producing cell 23 based on the classification result 75 is the subtype B, the prediction unit 68 selects the subtype B prediction model 78B. As shown in FIG. 10C, in a case where the subtype of the antibody-producing cell 23 based on the classification result 75 is the subtype C, the prediction unit 68 selects the subtype C prediction model 78C. The subtype A prediction model 78A in the case of FIG. 10A, the subtype B prediction model 78B in the case of FIG. 10B, and the subtype C prediction model 78C in the case of FIG. 10C are examples of a "matching tool" according to the technology of the present disclosure.

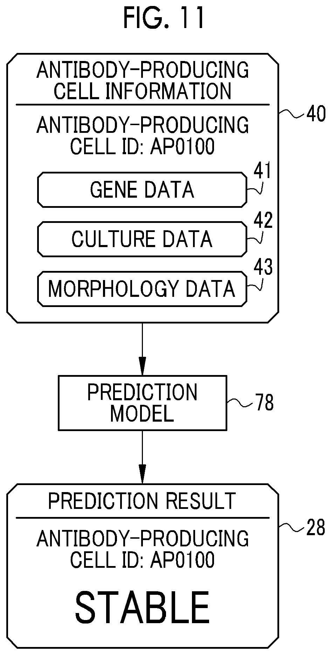

As shown in FIG. 11 as an example, the prediction unit 68 inputs the antibody-producing cell information 40 (the gene data 41, the culture data 42, and the morphological data 43) to the prediction model 78 selected as shown in FIG. 10, and causes the prediction model 78 to output a prediction result 28. The prediction model 78 is a machine learning model that has been trained to perform the production stability prediction based on the antibody-producing cell information 40. The training data of the prediction model 78 is a pair of the antibody-producing cell information 40 of the antibody-producing cell 23 generated in the past and the production stability (correct answer data) clarified by actually performing the production stability test on the clone 24 of the antibody-producing cell 23. The training data of the subtype A prediction model 78A is composed of only a pair of the antibody-producing cell information 40 and the production stability of the antibody-producing cell 23 of the subtype A generated in the past. Similarly, the training data of the subtype B prediction model 78B is composed of only a pair of the antibody-producing cell information 40 and the production stability of the antibody-producing cell 23 of the subtype B generated in the past. In addition, the training data of the subtype C prediction model 78C is composed of only a pair of the antibody-producing cell information 40 and the production stability of the antibody-producing cell 23 of the subtype C generated in the past. It is preferable that the prediction model 78 is a model that robustly predicts the production stability for the antibody-producing cell 23 that produces an unknown antibody 25. That is, it is preferable that the prediction model 78 is a domain-generalizable model whose domain is defined as a substance that serves as an active ingredient of a biopharmaceutical, such as the antibody 25.

It is noted that as in the case of the classification model 61, by inputting a plurality of elements constituting the antibody-producing cell information 40 excluded one or a plurality of the elements to the prediction model 78, outputting the prediction result 28 for training from the prediction model 78, and comparing the classification result in a case where the element is excluded with the prediction result 28 in a case where the element is not excluded, a contribution degree of the excluded element to the prediction result 28 may be calculated, and the element with high contribution degree may be narrowed down and used as the element finally input to the prediction model 78. For example, for the gene data 41 of the antibody-producing cell information 40, the gene expression level of a few to several tens of genes having a high contribution degree may be narrowed down to the gene expression level finally input to the prediction model 78 from the gene expression levels of hundreds of genes. In this case, the genes that exhibit a statistically significant difference in the gene expression level in terms of whether the production stability is stable or unstable may be narrowed down first, and then the narrowing down may be performed based on the contribution degree.

The prediction model 78 outputs, for example, a probability that the production stability of the antibody 25 of the antibody-producing cell 23 is stable and a probability that the production stability is unstable, and adopts the one having a higher probability as the prediction result 28. The probability that the production stability of the antibody 25 of the antibody-producing cell 23 is stable and the probability that the production stability is unstable sum to 100%.

Here, the elements of the antibody-producing cell information 40 that are input to the classification model 61 and the elements of the antibody-producing cell information 40 that are input to the prediction model 78 may be identical in their entirety, or some or all of the elements may differ. Similarly, the elements of the antibody-producing cell information 40 that are input to the subtype A prediction model 78A, the subtype B prediction model 78B, and the subtype C prediction model 78C may be identical in their entirety, or some or all of the elements may differ. In any case, it is preferable to acquire all the elements of the antibody-producing cell information 40 at once to save time.

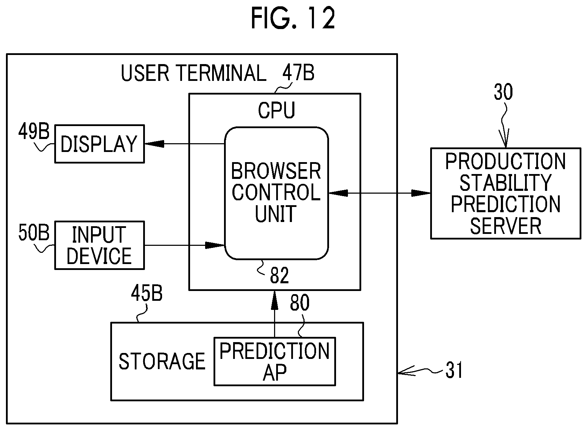

As shown in FIG. 12 as an example, a prediction AP 80 is stored in the storage 45B of the user terminal 31. The prediction AP 80 is installed in the user terminal 31 by the user U. The prediction AP 80 is an AP for performing the production stability prediction. In a case where the prediction AP 80 is activated, a CPU 47B of the user terminal 31 functions as a browser control unit 82 in cooperation with the memory 46 and the like. The browser control unit 82 controls an operation of a dedicated web browser of the prediction AP 80.

The browser control unit 82 reproduces various screens based on various screen data from the production stability prediction server 30, and displays the reproduced various screens on the display 49B. In addition, the browser control unit 82 receives various operation instructions input by the user U from the input device 50B through various screens. The browser control unit 82 transmits various requests corresponding to the operation instructions including the prediction request 33 to the production stability prediction server 30.

In a case where the prediction AP 80 is activated, the information input screen 85 shown in FIG. 13 as an example is displayed on the display 49B under the control of the browser control unit 82. An input box 86 for the antibody-producing cell information 40 for each clone 24 is provided on the information input screen 85. A file of the antibody-producing cell information 40 can be dropped into the input box 86. The input box 86 can be added by selecting an addition button 87A and 87B at the bottom. The addition button 87A is a button for adding one input box 86, and the addition button 87B is a button for adding 10 input boxes 86.

The user U selects a prediction button 88 after inputting desired antibody-producing cell information 40 into the input box 86. In a case where the prediction button 88 is selected, the browser control unit 82 generates the prediction request 33 including the antibody-producing cell information set 34 that is a collection of the antibody-producing cell information 40 input to the input box 86, and transmits the generated prediction request 33 to the production stability prediction server 30.

In addition, in a case where the production stability prediction is performed in the production stability prediction server 30, the prediction result display screen 95 shown in FIG. 14 as an example is displayed on the display 49B under the control of the browser control unit 82. A list table 96 in which the prediction result 28 and the classification result 75 for each clone 24 are summarized is displayed on the prediction result display screen 95. As described above, the prediction result 28 is presented to the user U in the form of delivery of screen data.

A save button 97 and an OK button 98 are provided in a lower portion of the prediction result display screen 95. In a case where the save button 97 is selected, the content of the list table 96 is stored in the storage 45B of the user terminal 31. In a case where the OK button 98 is selected, the display of the prediction result display screen 95 is erased.

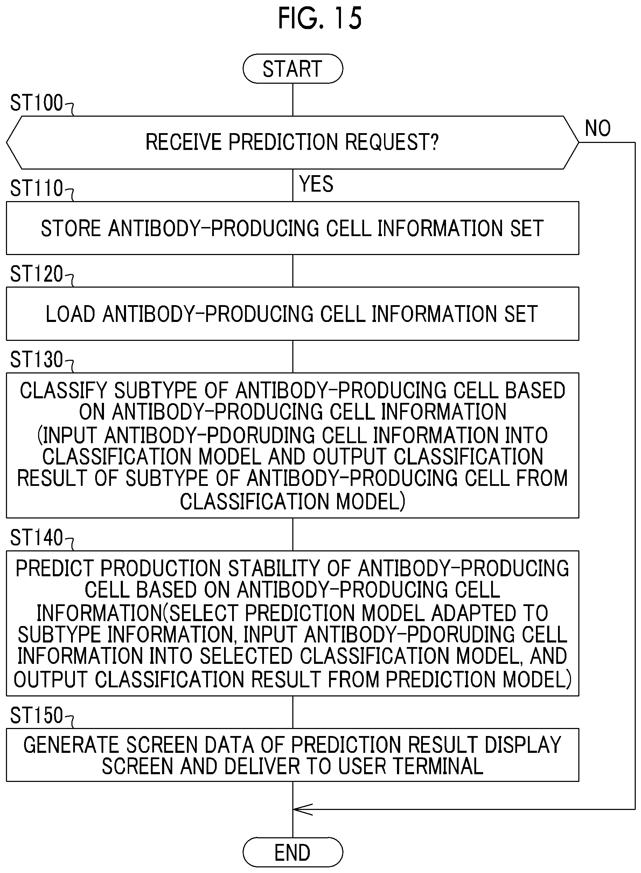

Next, an operation of the configuration described above will be described with reference to the flowchart shown in FIG. 15 as an example. In a case where the operation program 60 is activated in the production stability prediction server 30, the CPU 47A of the production stability prediction server 30 functions as the request reception unit 65, the RW control unit 66, the classification unit 67, the prediction unit 68, and the screen delivery control unit 69 as shown in FIG. 7. In addition, in a case where the prediction AP 80 is activated in the user terminal 31, the CPU 47B of the user terminal 31 functions as the browser control unit 82 as shown in FIG. 12.

The information input screen 85 shown in FIG. 13 is displayed on the display 49B of the user terminal 31 under the control of the browser control unit 82. In a case where the user U inputs the desired antibody-producing cell information 40 into the input box 86 and selects the prediction button 88 on the information input screen 85, the prediction request 33 is transmitted from the browser control unit 82 to the production stability prediction server 30. As shown in FIG. 1, the prediction request 33 includes the antibody-producing cell information set 34 that is a collection of the antibody-producing cell information 40, and the terminal ID of the user terminal 31 or the like.

In the production stability prediction server 30, the antibody-producing cell information 40 of the antibody-producing cell information set 34 included in the prediction request 33 is acquired by receiving the prediction request 33 in the request reception unit 65 (YES in Step ST100). The antibody-producing cell information set 34 is output from the request reception unit 65 to the RW control unit 66, and is stored in the storage 45A under the control of the RW control unit 66 (Step ST110). In addition, the terminal ID of the user terminal 31 included in the prediction request 33 is output from the request reception unit 65 to the screen delivery control unit 69.

The antibody-producing cell information set 34 is read out from the storage 45A by the RW control unit 66 (Step ST120). The antibody-producing cell information set 34 is output from the RW control unit 66 to the classification unit 67 and the prediction unit 68. The RW control unit 66 reads out the classification model 61 from the storage 45A and outputs the read classification model 61 to the classification unit 67. Furthermore, the prediction model set 62 is read out from the storage 45A by the RW control unit 66, and the readout prediction model set 62 is output to the prediction unit 68.

As shown in FIG. 8, in the classification unit 67, the antibody-producing cell information 40 is input to the classification model 61. As a result, the classification result 75 of the subtype of the antibody-producing cell 23 is output from the classification model 61 (Step ST130). The classification of the subtype of the antibody-producing cell 23 based on the antibody-producing cell information 40 is performed on the antibody-producing cell 23 constituting each clone 24. The classification result set 72 that is a collection of the classification results 75 of the antibody-producing cell 23 constituting each clone 24 is output from the classification unit 67 to the prediction unit 68 and the screen delivery control unit 69.

As shown in FIG. 10, in the prediction unit 68, one prediction model 78 adapted to the classification result 75 is selected from among the subtype A prediction model 78A, the subtype B prediction model 78B, and the subtype C prediction model 78C. Then, as shown in FIG. 11, the antibody-producing cell information 40 is input to the selected prediction model 78. As a result, the prediction result 28 is output from the prediction model 78 (Step ST140). The prediction of the production stability of the antibody-producing cell 23 based on the antibody-producing cell information 40 is performed on the antibody-producing cell 23 constituting each clone 24. The prediction result set 35 that is a collection of the prediction results 28 of the antibody-producing cell 23 constituting each clone 24 is output from the prediction unit 68 to the screen delivery control unit 69.

The screen delivery control unit 69 generates screen data of the prediction result display screen 95 shown in FIG. 14 based on the prediction result set 35 and the classification result set 72. The screen data of the prediction result display screen 95 is delivered to the user terminal 31 that is the transmission source of the prediction request 33 under the control of the screen delivery control unit 69 (Step ST150).

In the user terminal 31, the screen data of the prediction result display screen 95 is reproduced under the control of the browser control unit 82, and the reproduced prediction result display screen 95 is displayed on the display 49B. As a result, the prediction result 28 is presented to the user U.

As described above, the CPU 47A of the production stability prediction server 30 includes the classification unit 67 and the prediction unit 68. The classification unit 67 acquires the classification result 75 representing the subtype of the antibody-producing cell 23 derived from the subtype of the host cell 20 by generating the classification result 75. The prediction unit 68 performs the production stability prediction according to the classification result 75. Therefore, it is possible to improve the prediction accuracy of the production stability of the antibody-producing cell 23 as compared to a case where the subtype is not considered.

The request reception unit 65 acquires the antibody-producing cell information 40 related to the antibody-producing cell 23. The classification unit 67 classifies the subtype of the antibody-producing cell 23 based on the antibody-producing cell information 40, and acquires the classification result 75 of the subtype of the antibody-producing cell 23 as the subtype information. Therefore, it is possible to save the user U from the trouble of classifying the subtype based on the antibody-producing cell information 40 and inputting the classified subtype to the production stability prediction server 30 through the user terminal 31.

The classification unit 67 inputs the antibody-producing cell information 40 to the classification model 61, and causes the classification model 61 to output the classification result 75 of the subtype. Therefore, the classification result 75 of the subtype can be easily obtained.

The prediction unit 68 performs the production stability prediction based on the antibody-producing cell information 40. More specifically, the prediction unit 68 inputs the antibody-producing cell information 40 to the prediction model 78, and causes the prediction model 78 to output the prediction result 28 of the production stability. Therefore, the classification result 75 of the subtype can be easily obtained.

The antibody-producing cell information 40 includes the gene data 41 of the antibody-producing cell 23, the culture data 42 of the antibody-producing cell 23, and the morphological data 43 of the antibody-producing cell 23. The gene data 41, the culture data 42, and the morphological data 43 are very useful data for knowing the characteristics of the antibody-producing cell 23. Therefore, the classification accuracy of the subtype of the classification model 61, and the prediction accuracy of the production stability of the prediction model 78 can be improved. The antibody-producing cell information 40 may include at least one of the gene data 41, the culture data 42, or the morphological data 43.

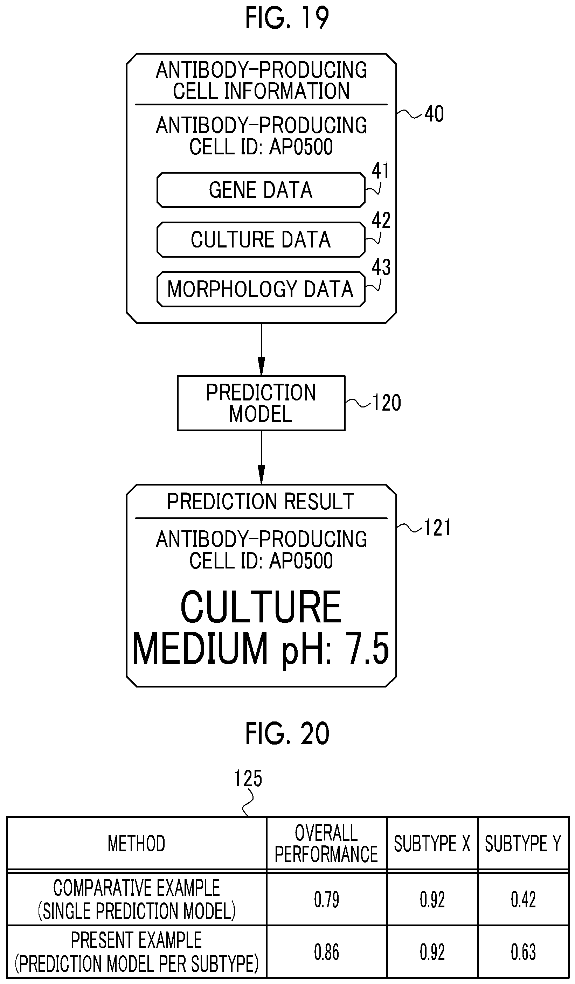

The prediction unit 68 selects the prediction model 78 adapted to the classification result 75 from among the subtype A prediction model 78A, the subtype B prediction model 78B, and the subtype C prediction model 78C, and performs the production stability prediction using the selected prediction model 78. By performing the production stability prediction using the prediction model 78 specialized for each subtype in this way, it is possible to further improve the prediction accuracy of the production stability of the antibody-producing cell 23 as compared to a case in which the production stability prediction is performed using one prediction model regardless of the subtype (see FIG. 20).

The screen delivery control unit 69 presents the prediction result 28 of the production stability of the antibody-producing cell 23 to the user U by delivering the screen data of the prediction result display screen 95 to the user terminal 31. The user U can easily perform the second selection of Step ST40 shown in FIG. 3 by referring to the prediction result 28.

In addition, the screen delivery control unit 69 presents the classification result 75 of the subtype of the antibody-producing cell 23 to the user U by delivering the screen data of the prediction result display screen 95 to the user terminal 31. The user U can also know the subtype in addition to the prediction result 28. The user U can perform various responses according to the subtype, such as setting the culture condition in the subsequent process development step 12 and the like in accordance with the subtype.

The host cell 20 that is the host cell is a mammalian-derived cell. The mammalian-derived cell is widely used in the manufacturing of the antibody pharmaceutical 10. Therefore, the general-purpose properties of the technology of the present disclosure can be improved.

In the present embodiment, the antibody-producing cell 23 that produces the antibody 25 that serves as the active ingredient of the antibody pharmaceutical 10 is the target cell. The antibody pharmaceutical 10 including the antibody 25 as the active ingredient is widely used not only for the treatment of chronic diseases, such as cancer, diabetes, and rheumatoid arthritis, but also for the treatment of rare diseases, such as hemophilia and a Crohn's disease. Therefore, according to the present embodiment in which the substance is the antibody 25, it is possible to promote the manufacturing of antibody pharmaceutical 10 widely used for the treatment of various diseases.

Then, the prediction unit 68 predicts the production stability of the antibody 25 as the characteristics of the target cell. Therefore, it is possible to save the trouble of the production stability test, and to greatly promote the manufacturing of the antibody pharmaceutical 10.

It is noted that the substance that serves as the active ingredient of the biopharmaceutical is not limited to the exemplified antibody 25. The substance may be an antibody-like protein, a peptide, a virus, or the like. In addition, examples of the cell product include cytokine (interferon, interleukin, or the like), hormone (insulin, glucagon, follicle-stimulating hormone, erythropoietin, or the like), a growth factor (insulin-like growth factor (IGF)-1, basic fibroblast growth factor (bFGF), or the like), a blood coagulation factor (seventh factor, eighth factor, ninth factor, or the like), an enzyme (lysosomal enzyme, deoxyribonucleic acid (DNA) degrading enzyme, or the like), a fragment crystallizable (Fc) fusion protein, a receptor, albumin, and a protein vaccine. Examples of the antibody 25 include a bispecific antibody, an antibody-drug conjugate, a low-molecular-weight antibody, and a sugar-chain-modified antibody.

### [Second embodiment]

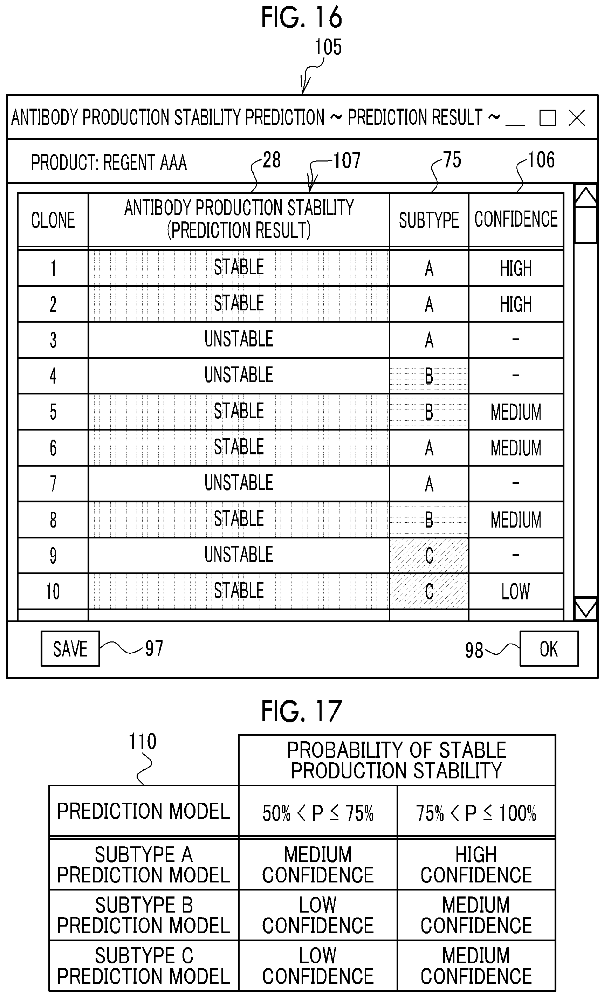

In the second embodiment, the screen delivery control unit 69 delivers a prediction result display screen 105 shown in FIG. 16 as an example to the user terminal 31 that is the transmission source of the prediction request 33. The prediction result display screen 105 is displayed on the display 49B under the control of the browser control unit 82. In addition to the prediction result 28 and the classification result 75, a list table 107 including a reliability degree 106 of the prediction result 28 is displayed on the prediction result display screen 105.

The reliability degree 106 is assigned by the prediction unit 68 only to the prediction result 28 indicating that the production stability is stable. The prediction unit 68 derives the reliability degree 106 based on a table 110 shown in FIG. 17 as an example. As shown in Table 110, the reliability degree 106 is determined by a combination of a type of the prediction model 78 used and a probability P that the production stability output by the prediction model 78 is stable. The reliability degree 106 has three stages of high, medium, and low. Then, the reliability degree 106 of the subtype A prediction model 78A is set to be relatively higher than the reliability degree 106 of the subtype B prediction model 78B and the reliability degree 106 of the subtype C prediction model 78C. For example, in a case where the prediction model 78 used is the subtype A prediction model 78A and the probability P is greater than 75% and 100% or less (75% < P ≤ 100%), the reliability degree 106 is high. In addition, in a case where the prediction model 78 used is the subtype B prediction model 78B or the subtype C prediction model 78C and the probability P is greater than 50% and 75% or less (50% < P ≤ 75%), the reliability degree 106 is low.

It is noted that as a reason why the reliability degree 106 of the subtype A prediction model 78A is set to be relatively higher than the reliability degree 106 of the subtype B prediction model 78B and the reliability degree 106 of the subtype C prediction model 78C, for example, the following can be considered. That is, the number of pieces of training data of the subtype B prediction model 78B and the subtype C prediction model 78C is smaller than the number of pieces of training data of the subtype A prediction model 78A, and the training of the subtype B prediction model 78B and the subtype C prediction model 78C is insufficient.

As described above, in the second embodiment, the screen delivery control unit 69 presents the reliability degree 106 of the prediction result 28 to the user U. The user U can perform the second selection by referring to not only the prediction result 28 but also the reliability degree 106. For example, in a case of FIG. 16, the prediction result 28 indicates that the production stability of all of the clones 1, 2, 5, 6, 8, and 10 is stable, but the clones 1 and 2 having a high reliability degree 106 can be prioritized for selection. In addition, for the clones having a low reliability degree 106, the production stability can be confirmed by actually performing the production stability test.

It is noted that the reliability degree 106 may be simply set to be high in a case where the prediction model 78 used is the subtype A prediction model 78A, set to be medium in a case where the prediction model 78 used is the subtype B prediction model 78B, and set to be low in a case where the prediction model 78 used is the subtype C prediction model 78C, without considering the probability P that the production stability output by the prediction model 78 is stable.

### [Third embodiment]

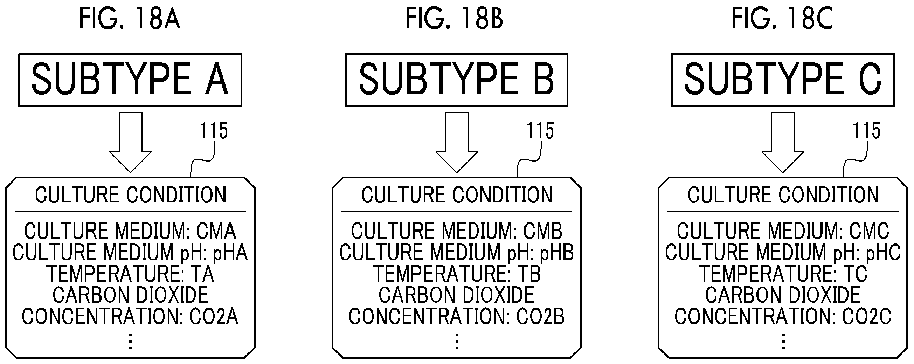

In the third embodiment, as shown in FIG. 18 as an example, the prediction unit 68 determines a basic culture condition 115 of the antibody-producing cell 23 in the process development step 12 and the GMP manufacturing step 13 after the clone generation step 11 based on the subtype information indicated by the classification result 75. The culture condition 115 includes a type of a culture medium, a hydrogen ion exponent (potential hydrogen: pH) of the culture medium, a temperature of a culture environment, a carbon dioxide concentration of the culture environment, and the like. The culture condition 115 is set in advance according to the subtype and is stored in the storage 45A. FIG. 18A shows the culture condition 115 in a case of the subtype A, FIG. 18B shows the culture condition 115 in a case of the subtype B, and FIG. 18C shows the culture condition 115 in a case of the subtype C. The culture condition 115 is displayed in response to the selection of the display button on the prediction result display screen 95 or 105, and can be viewed by the user U.

As described above, in the third embodiment, the prediction unit 68 determines the culture condition 115 of the antibody-producing cell 23 after the clone generation step 11 based on the subtype information. Therefore, it is possible to save the user U from the trouble of determining the culture condition 115. In addition, the subsequent steps can be performed under the culture condition 115 suitable for the antibody-producing cell 23 of each subtype. The culture condition 115 is merely a basic condition, and is a condition on the premise that various fine adjustments are made in the process development step 12 and the GMP manufacturing step 13.

### [Fourth embodiment]

In each of the above-described embodiments, the production stability of the antibody 25 is predicted as the characteristics of the antibody-producing cell 23, but the present disclosure is not limited to this. As shown in a prediction model 120 and a prediction result 121 of FIG. 19 as an example, the culture condition of the antibody-producing cell 23 after the clone generation step 11, here, the hydrogen ion exponent of the culture medium, may be predicted as the characteristics. In this case, the prediction unit 68 determines the culture condition of the antibody-producing cell 23 after the clone generation step 11 by predicting the culture condition of the antibody-producing cell 23.

As described above, in the fourth embodiment, the prediction unit 68 predicts the culture condition of the antibody-producing cell 23 as the characteristics of the antibody-producing cell 23. Therefore, it is possible to save the user U from the trouble of determining the culture condition. In addition, the subsequent steps can be performed under the culture condition suitable for the antibody-producing cell 23.

It is noted that the characteristics of the antibody-producing cell 23 to be predicted may be an amount of the antibody 25 produced, a quality of the antibody 25, or the like.

### Examples

In the present example, the host cell was a CHO cell, and the substance was an antibody. As evaluation samples, a plurality of clones of CHO cells that produce five types of antibodies were prepared. It was identified that the CHO cells had two types of subtypes, and hereinafter, the two types of subtypes are referred to as a subtype X and a subtype Y. After the specification testing was completed, the gene expression level of all genes of each sample was measured by RNA sequence analysis. It is noted that the specification testing was performed by suspension culture in a flask of 40 mL with a seeding number of clones of 5 × 10⁵ cells/mL.

In the prediction model, a logistic regression model that performs two-class classification of whether the production stability was stable or unstable, as an example, using the gene expression level of 100 genes or the like as explanatory variables, was prepared for each of the subtypes X and Y. In a case of training the prediction model, five-fold cross-validation was performed. In addition, the number of types of genes that exhibit a statistically significant difference in the gene expression level in terms of whether the production stability was stable or unstable was narrowed down to 300 to 400, and the number of the types of genes finally input by narrowing down based on the contribution degree was set to 100.

As a comparative example, one prediction model for performing the production stability prediction without distinguishing between the subtypes X and Y was prepared. In a case of training the prediction model of the comparative example, five-fold cross-validation was performed. In both the example and the comparative example, in the five-fold cross-validation, the pairs of the gene expression level and the like and the correct answer data of the production stability were divided for each of the five types of antibodies prepared as the evaluation samples, and the prediction accuracy of the prediction model was evaluated using the pair of the gene expression level and the like and the correct answer data of the production stability of the untrained antibody as the test data. More specifically, the pairs of the gene expression level and the like and the correct answer data of the production stability of four types of antibodies was used as the training data, and the pair of the gene expression level and the like and the correct answer data of the production stability of the remaining one type of antibody was used as the test data.

The results of the performance evaluation of the prediction models of the present example and the comparative example are shown in the table 125 of FIG. 20. The numerical value of the table 125 is an Area Under the Precision-Recall Curve (PR-AUC) that can comprehensively evaluate the performance of the machine learning model.

According to the table 125, the overall performance of the prediction model of the present example was higher than that of the prediction model of the comparative example. As a reason for this, it was considered that the prediction model of the comparative example had relatively low performance for the subtype Y, and the prediction accuracy of the production stability in a case of the subtype Y was relatively low, whereas the prediction model of the present example had significantly improved performance for the subtype Y as compared with the comparative example, and the prediction accuracy of the production stability in a case of the subtype Y was relatively high.

In a case where the prediction model of the comparative example was used, in a case where the subtype of the clone to be used for the production stability prediction was almost the subtype Y for some reason, there was a high possibility that the second selection would be erroneously performed by trusting the prediction result. On the other hand, according to the prediction model of the present example, the possibility of erroneously performing the second selection can be reduced.

The performance of both the prediction model of the present example and the prediction model of the comparative example for the subtype X is the same. That is, there is no superiority or inferiority between the prediction model of the present example and the prediction model of the comparative example in terms of the performance for the subtype X. This result indicates that it is not a problem to use the prediction model of the comparative example as the prediction model for the subtype X of the present example, and further indicates that it is not a problem to use the training data of the clone of the subtype Y as the training data of the prediction model for the subtype X of the present example. That is, it is shown that the prediction model of the present example has high flexibility.

As described above, it was confirmed that, according to the technology of the present disclosure, it is possible to improve the prediction accuracy of the characteristics of the cell.

One or a plurality of genes capable of classifying the subtype may be searched for, and the subtype may be classified based on the gene expression level of the gene. That is, the classification model 61 does not necessarily have to be used for the classification of the subtype. The prediction of the characteristics of the antibody-producing cell 23 does not necessarily have to use the prediction model 78. A rule-based method may be used instead of the classification model 61 and the prediction model 78.

The user U may be caused to input the subtype information through the user terminal 31, and the production stability prediction server 30 may acquire the subtype information input by the user U.

In each of the above-described embodiments, the prediction model is prepared for each subtype, but the present disclosure is not limited to this. For example, two prediction models of a subtype A prediction model and a prediction model for both the subtypes B and C may be prepared for the three subtypes of the subtypes A, B, and C. In addition, one prediction model may be used, and the input of the prediction model may include the subtype information.

The host cell 20 is not limited to the exemplified CHO cell. The host cell 20 may be a human embryonic kidney (HEK) cell. In addition, the host cell 20 is not limited to a mammalian-derived cell. The host cell 20 may be an insect cell.

The host cell is not limited to the exemplified host cell 20. The target cell is not limited to the exemplified antibody-producing cell 23. For example, an iPS cell may be used as the host cell, and a myocardial cell, a nerve cell, or the like induced to differentiate from the iPS cell may be used as the target cell. In this case, the characteristics of the target cell to be predicted are the difficulty of the differentiation induction, the proliferation ability of the cell induced to differentiation, the culture condition of the cell induced to differentiation after the differentiation induction, or the like.

The classification model 61 and the prediction model 78 may continue to be trained even after being stored in the storage 45A of the production stability prediction server 30.

The production stability prediction server 30 may be installed in a pharmaceutical company or a contract research organization, or may be installed in a data center independent on the pharmaceutical company or the contract research organization.

The screen data of the prediction result display screen 95 including the prediction result 28, the classification result 75, and the like may be delivered to the user terminal 31, or the prediction result 28 itself may be delivered to the user terminal 31. In this case, in the user terminal 31, the prediction result display screen 95 is generated based on the prediction result 28 and the like under the control of the browser control unit 82.

The method of presenting the prediction result 28 and the like to the user U is not limited to the presentation by the delivery of the exemplified screen data. The prediction result 28 and the like may be presented to the user U by printing the prediction result 28 and the like on a paper medium, or may be presented by attaching the prediction result 28 and the like to an electronic mail and transmitting the electronic mail to the user terminal 31.

The hardware configuration of the computer constituting the production stability prediction server 30 according to the technology of the present disclosure can be modified in various ways. For example, the production stability prediction server 30 may be configured using a plurality of physically separate computers as hardware, for the purpose of improving processing capability and reliability. For example, functions of the request reception unit 65 and the RW control unit 66 and functions of the classification unit 67, the prediction unit 68, and the screen delivery control unit 69 are provided in a distributed manner between two computers. In this case, the production stability prediction server 30 is configured using two computers.

As described above, the hardware configuration of the computer of the production stability prediction server 30 can be appropriately changed according to required performances, such as processing capacity, safety, and reliability. Not only the hardware but also the APs such as the operation program 60 may be duplicated or stored in a distributed manner between a plurality of storages for the purpose of securing safety and reliability.

In each of the embodiments, for example, as a hardware structure of a processing unit that executes various types of processing, such as the request reception unit 65, the RW control unit 66, the classification unit 67, the prediction unit 68, the screen delivery control unit 69, and the browser control unit 82, the following various processors can be used. The various processors include, for example, the CPUs 47A and 47B which are general-purpose processors executing software (the operation program 60 and the prediction AP 80) to function as various processing units as described above, a programmable logic device (PLD), such as a field programmable gate array (FPGA), which is a processor whose circuit configuration can be changed after manufacture, and a dedicated electric circuit, such as an application specific integrated circuit (ASIC), which is a processor having a dedicated circuit configuration designed to perform a specific process.

One processing unit may be configured by one of these various processors, or may be configured by a combination of two or more processors having the same type or different types (for example, a combination of a plurality of FPGAs and/or a combination of a CPU and an FPGA). A plurality of processing units may be configured by one processor.

As an example of configuring the plurality of processing units with one processor, first, there is a form in which one processor is configured by a combination of one or more CPUs and software and the processor functions as the plurality of processing units, as represented by computers such as a client and a server. A second example of the configuration is an aspect in which a processor that implements the functions of the entire system including a plurality of processing units using one integrated circuit (IC) chip is used. A representative example of the aspect is a system-on-chip (SoC). In this way, various processing units are configured by one or more of the above-described various processors as hardware structures.

Furthermore, specifically, an electric circuit (circuitry) obtained by combining circuit elements, such as semiconductor elements, can be used as the hardware structure of the various processors.

The technology according to the following appendices can be perceived from the above description.

### [Appendix 1]

A cell characteristic prediction apparatus that predicts characteristics of a target cell that is a cell obtained through monoclonalization from a host cell population that is a collection of host cells having a plurality of different subtypes, the cell characteristic prediction apparatus comprising a processor,
wherein the processor is configured to:
acquire subtype information representing a subtype of the target cell derived from a subtype of the host cell; and
perform prediction depending on the subtype information.

### [Appendix 2]

The cell characteristic prediction apparatus according to appendix 1,
wherein the processor is configured to:
acquire target cell information related to the target cell;
classify the subtype of the target cell based on the target cell information; and
acquire a classification result of the subtype of the target cell as the subtype information.

### [Appendix 3]

The cell characteristic prediction apparatus according to appendix 2,
wherein the processor is configured to:
input the target cell information to a first machine learning model; and
cause the first machine learning model to output the classification result of the subtype.

### [Appendix 4]

The cell characteristic prediction apparatus according to appendix 2 or 3,
wherein the target cell information includes at least one of gene data of the target cell, culture data of the target cell, or morphological data of the target cell.

### [Appendix 5]

The cell characteristic prediction apparatus according to any one of appendixes 1 to 4,
wherein the processor is configured to:
acquire target cell information related to the target cell; and
predict the characteristics of the target cell based on the target cell information.

### [Appendix 6]

The cell characteristic prediction apparatus according to appendix 5,
wherein the processor is configured to:
input the target cell information to a second machine learning model; and
cause the second machine learning model to output a prediction result of the characteristics of the target cell.

### [Appendix 7]

The cell characteristic prediction apparatus according to appendix 5 or 6,
wherein the target cell information includes at least one of gene data of the target cell, culture data of the target cell, or morphological data of the target cell.

### [Appendix 8]

The cell characteristic prediction apparatus according to any one of appendixes 1 to 7,
wherein a plurality of tools for predicting the characteristics of the target cell are provided, and
the processor is configured to:
   select a matching tool adapted to the subtype information from among the plurality of the tools; and
   perform the prediction using the matching tool.

### [Appendix 9]

The cell characteristic prediction apparatus according to any one of appendixes 1 to 8,
wherein the processor is configured to present a prediction result of the characteristics of the target cell to a user.

### [Appendix 10]

The cell characteristic prediction apparatus according to appendix 9,
wherein the processor is configured to present the subtype information to the user.

### [Appendix 11]

The cell characteristic prediction apparatus according to appendix 9 or 10,
wherein the processor is configured to present a reliability degree of the prediction result to the user.

### [Appendix 12]

The cell characteristic prediction apparatus according to any one of appendixes 1 to 11,
wherein the processor is configured to determine a subsequent culture condition of the target cell based on at least one of the subtype information or a prediction result of the characteristics of the target cell.

### [Appendix 13]

The cell characteristic prediction apparatus according to any one of appendixes 1 to 12,
wherein the host cell is a mammalian-derived cell.

### [Appendix 14]

The cell characteristic prediction apparatus according to any one of appendixes 1 to 13,
wherein the target cell is a cell that produces a substance that serves as an active ingredient of a biopharmaceutical, and
the processor is configured to predict production stability of the substance as the characteristics of the target cell.

### [Appendix 15]

The cell characteristic prediction apparatus according to appendix 14,
wherein the substance is an antibody.

### [Appendix 16]

The cell characteristic prediction apparatus according to any one of appendixes 1 to 15,
wherein the processor is configured to predict a culture condition of the target cell as the characteristics of the target cell.

The technology of the present disclosure can also be combined with various embodiments and/or various modification examples described above, as appropriate. The disclosed technology is not limited to the above embodiment and may adopt various configurations without departing from its gist. Furthermore, the technology of the present disclosure extends to a storage medium that non-transitorily stores the program, and a computer program product including the program, in addition to the program.

The above-described contents and the above-shown contents are the detailed description of the parts according to the technology of the present disclosure, and are merely an example of the technology of the present disclosure. For example, the above description of the configuration, the function, the operation, and the effect are the description of examples of the configuration, the function, the operation, and the effect of the parts according to the technology of the present disclosure. Accordingly, it goes without saying that unnecessary parts may be deleted, new elements may be added, or replacements may be made with respect to the above-described contents and the above-shown contents within a range that does not deviate from the gist of the technology of the present disclosure. In addition, in order to avoid complications and facilitate grasping the parts according to the technology of the present disclosure, in the above-described contents and the above-shown contents, the description of technical general knowledge and the like that do not particularly require description for enabling the implementation of the technology of the present disclosure are omitted.

In the present specification, "A and/or B" has the same meaning as "at least one of A or B". That is, "A and/or B" means that it may be only A, only B, or a combination of A and B. In addition, in the present specification, also in a case in which three or more matters are expressed in association by "and/or", the same concept as "A and/or B" is applied.

All of the documents, the patent applications, and the technical standards described in the present specification are incorporated herein by reference to the same extent as in a case in which each of the documents, patent applications, and technical standards is specifically and individually described by being incorporated by reference.

## Claims

1. A cell characteristic prediction apparatus that predicts characteristics of a target cell that is a cell obtained through monoclonalization from a host cell population that is a collection of host cells having a plurality of different subtypes, the cell characteristic prediction apparatus comprising a processor,
wherein the processor is configured to:
acquire subtype information representing a subtype of the target cell derived from a subtype of the host cell; and
perform prediction depending on the subtype information.

2. The cell characteristic prediction apparatus according to claim 1,
wherein the processor is configured to:
acquire target cell information related to the target cell;
classify the subtype of the target cell based on the target cell information; and
acquire a classification result of the subtype of the target cell as the subtype information.

3. The cell characteristic prediction apparatus according to claim 2,
wherein the processor is configured to:
input the target cell information to a first machine learning model; and
cause the first machine learning model to output the classification result of the subtype.

4. The cell characteristic prediction apparatus according to claim 2,
wherein the target cell information includes at least one of gene data of the target cell, culture data of the target cell, or morphological data of the target cell.

5. The cell characteristic prediction apparatus according to claim 1,
wherein the processor is configured to:
acquire target cell information related to the target cell; and
predict the characteristics of the target cell based on the target cell information.

6. The cell characteristic prediction apparatus according to claim 5,
wherein the processor is configured to:
input the target cell information to a second machine learning model; and
cause the second machine learning model to output a prediction result of the characteristics of the target cell.

7. The cell characteristic prediction apparatus according to claim 5,
wherein the target cell information includes at least one of gene data of the target cell, culture data of the target cell, or morphological data of the target cell.

8. The cell characteristic prediction apparatus according to claim 1,
wherein a plurality of tools for predicting the characteristics of the target cell are provided, and
the processor is configured to:
select a matching tool adapted to the subtype information from among the plurality of tools; and
perform the prediction using the matching tool.

9. The cell characteristic prediction apparatus according to claim 1,
wherein the processor is configured to present a prediction result of the characteristics of the target cell to a user.

10. The cell characteristic prediction apparatus according to claim 9,
wherein the processor is configured to present the subtype information to the user.

11. The cell characteristic prediction apparatus according to claim 9,
wherein the processor is configured to present a reliability degree of the prediction result to the user.

12. The cell characteristic prediction apparatus according to claim 1,
wherein the processor is configured to determine a subsequent culture condition of the target cell based on at least one of the subtype information or a prediction result of the characteristics of the target cell.

13. The cell characteristic prediction apparatus according to claim 1,
wherein the host cell is a mammalian-derived cell.

14. The cell characteristic prediction apparatus according to claim 1,
wherein the target cell is a cell that produces a substance that serves as an active ingredient of a biopharmaceutical, and
the processor is configured to predict production stability of the substance as the characteristics of the target cell.

15. The cell characteristic prediction apparatus according to claim 14,
wherein the substance is an antibody.

16. The cell characteristic prediction apparatus according to claim 1,
wherein the processor is configured to predict a culture condition of the target cell as the characteristics of the target cell.

17. An operation method for a cell characteristic prediction apparatus that predicts characteristics of a target cell that is obtained through monoclonalization from a host cell population that is a collection of host cells having a plurality of different subtypes, the operation method comprising:
acquiring subtype information representing a subtype of the target cell derived from a subtype of the host cell; and
performing the prediction depending on the subtype information.

18. An operation program for a cell characteristic prediction apparatus that predicts characteristics of a target cell that is obtained through monoclonalization from a host cell population that is a collection of host cells having a plurality of different subtypes, the operation program causing a computer to execute a process comprising:
acquiring subtype information representing a subtype of the target cell derived from a subtype of the host cell; and
performing the prediction depending on the subtype information.
